# EUROPEAN PATENT APPLICATION

(11) **EP 4 840 964 A1**
(43) Date of publication of application: **18.11.2026**
(21) Application number: 25175642.5
(22) Date of filing: 12.05.2025
(51) Int. Cl.: C12N 15/85, C12N 15/86

(54) **CELL CULTURE METHOD AVOIDING CELL DENSITY EFFECTS**

(71) Applicant: Sartorius Stedim Biotech GmbH, 37079 Göttingen (DE)
(72) Inventor: Wagner, Nazgül, 37079 Göttingen (DE); Niemann, Julia, 37079 Göttingen (DE)
(74) Representative: Cohausz & Florack

(57) **Abstract**

The present disclosure provides a cell culture workflow that mitigates or avoids the negative impact of high cell density on transient expression to maintain cell specific productivity. This is achieved by a method for transiently producing a biological product in cell culture, comprising: providing a population of cells, wherein the population comprises the cells at a first concentration, introducing one or more nucleic acid molecules encoding the product into the cells, thereby generating a population of genetically modified cells, concentrating the population of genetically modified cells, thereby generating a population of concentrated cells comprising the genetically modified cells at a second concentration, wherein the second concentration is higher than the first concentration; and cultivating the concentrated cells under conditions conducive to expression of the product.

## Description

The present disclosure relates to the field of cell culture, particularly to methods for transient production of biological products in cell culture. More specifically, the present disclosure provides a cell culture workflow that mitigates or avoids the negative impact of high cell density on transient expression to maintain cell specific productivity.

### BACKGROUND OF THE INVENTION

Cell culture is a fundamental technique in biotechnology, medicine, and research, enabling the growth and maintenance of cells under controlled conditions outside their natural environment. It is widely used for applications such as protein production, gene therapy, drug discovery, and regenerative medicine. A critical factor influencing cell culture outcomes is cell density, which refers to the number of cells per unit volume of culture medium.

Impact of cell density is well-documented in the art and can significantly influence cell behavior, including proliferation, differentiation, and productivity. On the one hand, it is considered advantageous to increase cell densities when producing a specific biological product, e.g. viral vector or recombination protein, as this is often associated with an increased space-time yield of the culture. On the other hand, at high cell densities, cells may experience nutrient depletion, accumulation of waste products, and increased competition for space and resources. These conditions can lead to reduced cell viability, altered metabolic activity, and suboptimal production of desired biomolecules. Conversely, at low cell densities, cells may not receive sufficient signals from neighboring cells, which can also negatively affect growth and functionality. These density-dependent limitations are particularly problematic in processes where manufacturing efficiency is limited, such as viral vector manufacturing. Current cell culture methods often struggle to maintain optimal cell density throughout the culture period, leading to variability in productivity and quality.

In addition to these well-known effects of cell density in general, there is a particular challenge in bioprocesses based on transient expression termed "cell density effect" (CDE). CDE corresponds to a reduction of cell-specific productivity at increasing cell densities. This effect has been observed for various cell types, including mammalian, insect, and avian derived cell types used for production of biological products, e.g. viral vectors, via transient transfection. The causes for CDE are still not fully resolved; plausible explanations include the presence of inhibitory components in the cell culture medium, as well as the cell physiological conditions as driving factors of CDE.

Previous works have attempted to overcome cell density effects in cell culture using specific culture media and growth factors (WO 2023/247733 A1), specific bioreactor design and control of environmental conditions such as temperature (WO 2023/232922 A1).

Against this background, it is an object of the invention to provide a cell culture method, particularly a workflow for producing a biological product, that avoids the cell density effect but improve productivity of the cell culture. It is a further object of the invention to provide a cell culturing system by which the cell culturing method can be carried out, preferably in an at least partially automated fashion.

### SUMMARY OF THE INVENTION

The invention achieves the object by the features of the claims. In a first aspect, the invention solves the aforementioned problems by a method for transiently producing a biological product in cell culture, comprising:
a. providing a population of cells, wherein the population comprises the cells at a first concentration,
b. introducing one or more nucleic acid molecules encoding the product into the cells, thereby generating a population of genetically modified cells,
c. concentrating the population of genetically modified cells, thereby generating a population of concentrated cells comprising the genetically modified cells at a second concentration,
   wherein the second concentration is higher than the first concentration; and
d. cultivating the concentrated cells under conditions conducive to expression of the product.

In a second aspect, the invention solves the aforementioned problems by providing a cell culturing system configured to carry out a method according to the first aspect of the present invention, the system comprising:
- means for providing a population of cells capable of expressing the product, wherein the population comprises the cells at a first concentration,
- means for introducing one or more nucleic acid molecules encoding the product into the cells, thereby generating a population of genetically modified cells,
- means for concentrating the population of genetically modified cells, thereby generating a population of concentrated cells comprising the genetically modified cells at a second concentration,
   wherein the second concentration is higher than the first concentration;
- means for cultivating the cells under conditions conducive to expression of the product; and
- optionally means for harvesting the product.

In a third aspect, the invention solves the aforementioned problems by providing a use of a cell culturing system according to the second aspect in a method according to the first aspect.

It is to be understood that the features disclosed in relation to any particular aspect also pertain to any other aspect of the present invention.

The invention is particularly advantageous for manufacturing a biological product, e.g. a viral vector, by transient expression systems. Particularly, the invention addresses the cell density effect, wherein a drop of cell-specific productivity at high cell densities is observed. The invention mitigates the cell density effect by essentially performing the introduction of the nucleic acid molecule(s) for transient expression by providing a population of cells at a first concentration to generate a population of genetically modified cells, but cultivating the cells under conditions conducive to expression of the product at a second concentration which is higher than the first concentration. In particular, at the first concentration it is desirable to avoid the cell density effect, i.e. to obtain a population of genetically modified cells with high efficiency. Then, the cells are concentrated and undergo culture for production at the second concentration which is higher than the first concentration in order to efficiently utilize the reactor space, i.e., obtain a higher space-time yield, which is in turn indicative of an efficient utilization of reactor volume over time, resulting in increased productivity. The invention thereby maintains or increases cell-specific yields, despite delivering the population of genetically modified cells at high concentration (second concentration). This results in overall improved production time efficiency offering a robust and efficient solution for maintaining optimal cell culture conditions.

### DEFINITIONS

The following terms are used throughout the specification and claims in accordance with the definitions provided below.

In the context of the present disclosure, the term "cell culture" is intended to refer to the process of growing and maintaining cells under controlled conditions outside their natural environment, typically in a laboratory setting. This includes the cultivation of cells in a suitable growth medium, which provides essential nutrients, growth factors, and environmental conditions (e.g., temperature, pH, and gas exchange) to support cell survival, proliferation, and functionality. Although the methods disclosed herein are primarily intended for the culturing of eukaryotic cells, especially mammalian cells such as HEK or CHO cells, the methods and system disclosed herein may be adapted to other cell types such bacterial, yeast, avian, insect, and plant cells. Cultured cells are also referred to as "host cells" throughout the present disclosure.

In the context of the present disclosure, the term "transient production" or "transient expression" refers to a process in which at least one nucleic acid molecule encoding a biological product is introduced into host cells for temporary expression, without permanent genomic integration. Transient production may occur in fully transient expression systems, where all recombinant genes are introduced transiently, or in partially transient expression systems, where one or more genes are stably integrated into the host cell genome, while additional genes required for production are supplied transiently. For example, in the production of viral particles such as AAV or lentivirus, certain viral genes (e.g., helper functions or capsid proteins) may be introduced transiently, while others (e.g., packaging genes, regulatory elements) may be stably expressed in the host cells.

In the context of the present disclosure, the term "biological product" is intended to refer to any substance derived from the cell culture, which is used for therapeutic, diagnostic, or research purposes. Biological products include, but are not limited to, proteins, antibodies, enzymes, vaccines, gene therapies, viral vectors and nucleic acids.

In the context of the present disclosure, the term "population of cells" is intended to refer to a group of cells of the same or different types that are cultured together under defined conditions. Although it is preferred that the population consists of a homogeneous group of cells (e.g., a clonal cell line), the present invention can be routinely adapted to cell culture of a heterogeneous mixture of cells (e.g., primary cells or co-cultures). The term "population of cells" is intended to encompass cells at various stages of growth, differentiation, transfection or transduction, and includes genetically modified and unmodified cells.

In the context of the present disclosure, the terms "nucleic acid" and "nucleic acid molecule" are used interchangeably herein and are intended to refer to a molecule or construct comprising DNA, RNA, or synthetic analogs thereof, which is introduced into the cells in accordance with the invention, preferably by transfection. This includes, but is not limited to, plasmids, viral vectors, mRNA, CRISPR-Cas components, and other gene delivery systems designed to introduce genetic material into cells. Preferably, it includes one or more plasmids. The nucleic acid encodes one or more genes of interest, and may additionally encode regulatory elements, and/or markers for selection or tracking.

In the context of the present disclosure, the term "introducing one or more nucleic acid molecules" (also referred to herein as "genetic modification") is intended to refer to any method or process by which exogenous nucleic acid molecules are delivered into a cell or population of cells, such that the nucleic acid molecules are available for transcription and/or translation within the cellular environment. This includes, but is not limited to, chemical or physical transfection methods (such as lipofection or electroporation), viral-mediated delivery (transduction), and infection with wild-type or genetically modified viruses capable of transferring genetic material. Therein, it is generally preferred that the introduction is achieved via transient transfection, wherein the nucleic acid molecules are expressed episomally and are not intended to integrate into the host genome.

In the context of the present disclosure, the term "genetically modified cells", also referred to as "transfected cells" and/or "transduced cells", is intended to refer to cells that have been altered through the introduction of exogenous nucleic acids using molecular-biological techniques. In the context of the present invention, it is preferred that the cells are only transiently modified in that the exogenous nucleic acid molecule(s) is/are not intended to integrate into the genome of the cell.

In the context of the present disclosure, the term "conditions conducive to expression" is intended to refer to the environmental and cultural parameters that enable or enhance the transcription and translation of introduced nucleic acids in cells. These conditions include, but are not limited to, optimal temperature, pH, oxygen levels, nutrient availability, and the presence of inducers or activators that regulate gene expression. Such conditions are tailored to the specific requirements of the host cells and the nucleic acids being expressed.

### DETAILED DESCRIPTION

According to a first aspect, the present invention provides a method for transiently producing a biological product in cell culture.

The method according to the first aspect comprises, as a first step, providing a population of cells (also referred to as "cell population"). The cell population can be provided as part of a continuously maintained culture, such as a seed train or a master culture. For example, the continuously maintained culture may be kept in a shake flask, or may be kept in a bioreactor or perfusion system. It is understood that this step does not necessarily require an active action, because, for example, the cell population may be provided by accessing a pre-existing culture of cells. In some embodiments, the cell population is provided as part of an automated or semi-automated workflow, where the cells are transferred or inoculated into a production vessel without requiring manual handling.

In accordance with the present invention, the cells may be provided in any liquid suitable for cultivation and/or genetic modification of cells in a cell culture. Suitable liquids include, but are not limited to, buffered saline solutions (e.g.: PBS, HBSS), cell culture medium (e.g., DMEM, RPMI, or Opti-MEM, preferably serum-free, optionally containing transfection-enhancing additives such as polybrene), or specialized culture and expression media (e.g., Sf-900 III SFM, Opti-CHO, FreeStyle293).

According to step a. of the method, the cell population is provided at a first concentration. In the context of the present disclosure, the first concentration refers to a number of cells per volume of a liquid in which the cells are suspended. For example, a first concentration of 1 x 10⁶ cells per mL means that 1'000'000 cells are present in each milliliter of liquid, e.g. cell culture medium.

In an exemplary embodiment of the present disclosure, the population of cells is provided by providing a cell seed comprising the cells at a seeding concentration which optionally differs from the first concentration, and adjusting the cell concentration of the cell seed until the first concentration of the cells is reached. In this embodiment, it is preferred that the provision of the cells is accompanied by an exchange of cell culture media and/or a dilution in fresh cell culture media with the aim of removing any accumulated cell debris, extracellular vesicles and/or unwanted metabolites from the cells. Although the type of vessel harboring the cell seed is not critical for achieving the advantages associated with the present invention, it is further preferred that the cell seed is obtained from a continuous seed system, such as a perfusion-based seed train or from systems connected to devices enabling periodic centrifugation for medium exchange such as counterflow centrifugation (e.g., Ksep^{®}).

In some embodiment, the cell seed may be a continuous seed system, wherein cells are cultured in a perfusion culture to a high cell density. Once a part of the cells are removed and used for transient production, the remaining cell seed is scaled up again and therefore ready for another part of the culture to be aspirated for transient production.

The first concentration is generally assumed to be lower than the concentration at which the cells are maintained in a continuously maintained culture (also referred to as "maintained concentration") such as a cell seed but can also be higher than the maintained concentration. Accordingly, the step of providing the cell population may encompass diluting or concentrating steps in order to adjust the cell concentration until the first concentration is reached.

Therein, it is preferred that the first concentration is a concentration at which no cell density effect is present in the population of cells. As it is commonly appreciated in the field, such a concentration depends on a variety of factors including the cells (e.g., the cell line), the cell culture medium, the presence of additives, the culturing conditions, and the conditions by which nucleic acids are introduced into the cell for transient transfection. Generally, the cell density effect is characterized by an elevated cell density at which the cell-specific productivity significantly decreases compared to lower cell density. As illustrated in the examples below, the cell density effect led to a 4.1-fold decrease in capsid yield per cell when comparing a production performed at 3 million cells per mL to one performed at an elevated concentration of 9 million cells per mL. Hence, the cell density effect may be referred to as a significant reduction of cell specific productivity of a cell concentration compared to a lower cell concentration, e.g. a reduction in cell-specific productivity by 10% or more, or a reduction by 20% of more. As the skilled person readily understands, the cell density effect and the cell density at which it is observed depend on various factors, including host cell, culturing system, transfection system, biological product, state of culture, etc. The skilled person also knows methods on how to identify the cell density at which the cell density effect is observed, as also outlined in the examples herein.

For example, the first concentration may be in the range of 0.01-6 x 10⁶ cells per mL, preferably 0.5-6 x 10⁶ cells per mL, more preferably 1-3 x 10⁶ cells per mL, e.g. 3 x 10⁶ cells per mL. For example, in the case of HEK cells, the first concentration may preferably be in the range of 0.5-3 x 10⁶ cells per mL; in the case of CHO cells, the first concentration may preferably be in the range of 1-5 x 10⁶ cells per mL; in the case of insect cells, the first concentration may preferably be in the range of 1-4 x 10⁶ cells per mL.

In particular, cells may be sourced from a seed bioreactor containing a high concentration of cells (e.g., 20 x 10⁶ cells per mL), such that the providing step encompasses diluting the sourced cells down to reach the first concentration, optionally including an exchange of cell culture medium. Alternatively, cells may be sourced from a master culture being maintained in a shake flask at a medium or high cell concentration (e.g., 2-5 x 10⁶ cells per mL) such that a dilution may not be necessary to reach the first concentration. In this scenario, it is preferred that the step of providing the cells includes an exchange of cell culture medium to remove any unwanted metabolites and cell debris from the culture before introducing the one or more nucleic acid molecules.

In an exemplary embodiment according to the present disclosure, the cells are eukaryotic cells, preferably mammalian or insect cells, further preferably wherein the cells are selected from the group consisting of HEK and CHO cells, further preferably wherein the cells are HEK cells. The advantages of eukaryotic cells for the expression of biological products, especially of complex biological products such as proteins comprising glycosylation or complex folding patterns, are commonly known. Especially the mammalian cell lines HEK (Human Embryonic Kidney) and CHO (Chinese Hamster Ovary) are widely established vehicles for the production of biological products, as is the insect cell line Sf9. Other known cell lines compatible with the present invention include, but are not limited to, HeLa and BHK. Due to their compatibility with adenoviral helper functions, HEK cells are inherently compatible with the production of particles of adeno-associated virus (AAV), which is particularly preferred in the context of the present disclosure.

The method according to the first aspect of the present disclosure further comprises a step b. of introducing one or more nucleic acid molecules encoding the biological product into the cells provided at the first concentration, thereby generating a population of genetically modified cells (also referred to as "nucleic acid introduction" throughout the present disclosure).

In certain embodiments, the introducing in step b. is carried out by contacting the cells with a nucleic acid delivery composition under conditions that facilitate uptake and gene expression. For example, the delivery composition may include the one or more nucleic acid molecules in combination with a reagent capable of mediating cellular uptake (also referred to as "transfection reagent" throughout the present disclosure). Such reagents typically include cationic lipids, liposomes, polymers (e.g., polyethyleneimine), dendrimers, or lipid-based nanoparticles, which facilitate complex formation with the negatively charged nucleic acid molecules through electrostatic interactions.

The resulting nucleic acid-transfection reagent complexes, often referred to as transfection complexes, are typically incubated with the cells under conditions conducive to uptake, such as in serum-free or reduced-serum media, for a defined period, after which the medium may be replaced or supplemented to support continued cell viability and gene expression. This process leads to the formation of the genetically modified cells, in which the introduced nucleic acid molecule(s) is/are maintained, wherein a transient maintenance is preferred.

For example, the introduction may be performed using commercially available reagents such as Lipofectamine^{™} (Thermo Fisher Scientific), FuGENE^{®} (Promega), jetPEI^{®} (Polyplus), or similar cationic formulations known in the art. The choice of reagent, nucleic acid dose, complexation ratio, and incubation time may be optimized using routine measures based on the type of host cell, the nature of the nucleic acid, and the desired expression profile.

It is generally appreciated that the population of genetically modified cells may encompass the entire cell population, but - depending on the efficiency of the nucleic acid introduction - may encompass only a fraction of the cell population, whereas the remaining cells remain unmodified. It is preferred that the population of genetically modified cells is at least 60 %, more preferably at least 70 % or at least 80 % of the cell population, corresponding to a transfection efficiency of 60 %, 70 % or 80 %, respectively.

Any mode of introducing the one or more nucleic acid molecules into the host cells can be used with the present invention. Exemplary nucleic acid introduction methods include, but are not limited to, viral transduction, plasmid-based transfection (e.g. using liposomes or polymeric agents), and electroporation.

The one or more nucleic acid molecules (also referred to as "nucleic acids" or "nucleic acid molecule(s)" throughout the present disclosure), optionally in admixture or combination with a transfection reagent, may be introduced into the host cell culture using various methods. Introduction may be achieved by pipetting, direct addition via automated liquid handling systems, dropwise dispensing, or by spraying or nebulization for fine distribution. In some embodiments, electroporation may be employed, in which cells and nucleic acids are combined in a specialized cuvette or flow system before being returned to the culture. In some embodiments, the nucleic acid molecule(s) may be introduced to the entire culture volume, or alternatively to a portion of the culture, such as a concentrated region near the cells, followed by gentle mixing or diffusion. Depending on the method used, shaking conditions may be adjusted to facilitate homogeneous distribution while avoiding excessive shear stress. The temperature at the time of nucleic acid introduction may be ambient or controlled to enhance transfection efficiency, typically ranging from 4°C (for electroporation) to 37°C (for chemical transfection or viral transduction), depending on the specific protocol.

In particular, adding nucleic acids directly into a bioreactor containing a cell culture is considered to fulfil both step a. of providing the population of cells and step b. of introducing nucleic acids to the population of cells. Hence, steps a. and b. may be performed sequentially or in parallel.

It is generally appreciated that the step of introducing one or more nucleic acid molecules encompasses introducing multiple nucleic acid constructs into the cell population, such as two or more different plasmids, and/or two or more different AAV particles.

It is preferred that the one or more nucleic acid molecules encode a viral vector, preferably selected from the group consisting of adenoviral vector, lentiviral vector, AAV vector, baculoviral vector, HSV vector, vaccinia virus vector, and SFV vector. Of this group, AAV vectors, adenoviral vectors and lentiviral vectors are of particular relevance. It is especially preferred in accordance with the present invention that the nucleic acid molecule(s) encode a recombinant AAV vector (rAAV). AAV vectors are widely used in gene therapy to deliver therapeutic genes to target tissues, and also find use as vehicles for antigens in vaccine development.

In accordance with an exemplary embodiment of the present disclosure, it is preferred that the biological product is an intact virus, wild-type or recombinant, capable of infecting a target cell, preferably wherein said virus retains its full structural and functional integrity, including its capsid and a polynucleotide. The research leading up to the present invention showed that the method allows for the consistent, reproducible and scalable production of functional AAV particles. The method minimizes metabolic stress, leading to healthier cells and higher AAV yields. The method further allows for high transfection efficiency and leads to high-quality product suitable for all downstream applications.

Without wishing to be bound by theory, it is presently believed that the cell density effect (CDE) affects the yield of cell cultures producing functional AAV particles following transient transfection for the following reasons: 1) Inhibitory compounds and extracellular vesicles accumulating throughout culture and increasing over cell density. 2) Changes in the cell physiology at elevated cell densities. 3) Possibly, the transfection complexes have to be prepared under suboptimal conditions, namely at high concentrations due to a high number of cells, which likely leads to the transfection complexes having a suboptimal size for transfection, which in turn contributes to a decrease in yield.

In some embodiments, the biological product may contain by-products or impurities, including but not limited to incorrectly folded protein or non-functional AAV particles. Such by-products and impurities need to be removed in downstream processing of the biological product. It is, however, an advantage of the method according to the first aspect of the present disclosure that the quality of the biological product is increased and/or the amount of impurities in the biological product is reduced due to a reduced influence of CDE on the cell culture. For example, biological products produced according to the present invention may have an improved quality of glycosylation patterns and/or protein folding, or an improved viral capsid integrity and/or viral packaging efficiency.

In an exemplary embodiment, prior to step b., the population of cells is maintained for a period of time. This is intended to allow for the cells to adapt to the conditions accompanying the first concentration, i.e. the lack of CDE-associated disadvantages. Without wishing to be bound by theory, it is believed that adjusting the cell concentration to reach the first concentration may restore the cells' access to nutrients, reestablish optimal oxygen levels within the culture medium, and reestablish normal cell-cell interactions. As a consequence, it is believed that the maintaining period permits the downregulation of stress response pathways and resumption of cell cycle progression, which may both aid in improving transfection efficiency and support higher production rates and increased product quality. Therein, it is preferred that the period of time is at least 10 minutes, at least 15 minutes, at least 20 minutes, at least 25 minutes, at least 30 minutes, e.g. up to 4 hours, up to 2 hours or up to 1 hour.

Following the nucleic acid introduction step, the host cells may be maintained under resting conditions for a defined period before the culture resumes and step c. of the method is carried out. During this period, active agitation or perfusion may be reduced or halted to minimize mechanical stress and allow optimal uptake and processing of the introduced nucleic acid molecule(s). Resting conditions may involve incubating the cells in a static or low-shear environment, optionally in the presence of specific buffering agents or protective additives that support cell viability and transgene expression. The duration of the resting phase may vary depending on the cell type and nucleic acid introduction method, typically ranging from a few minutes to several hours. In some embodiments, temperature adjustments, such as transient cooling or incubation at sub-physiological temperatures (e.g., 30-35°C), may be applied to enhance stability and improve transgene expression kinetics. After the resting period, standard culture conditions, including agitation, aeration, and nutrient exchange, are resumed to support continued cell growth and expression of the biological product. Accordingly, in an exemplary embodiment, at the end of step b. and prior to step c., the cells are incubated for a period of time, throughout which the cells become genetically modified by taking up the exogenous nucleic acid molecule(s). It is preferred that the period of time is at least 10 minutes, at least 15 minutes, at least 20 minutes, at least 25 minutes, at least 30 minutes, e.g. up to 4 hours, up to 2 hours or up to 1 hour, a period of 4 hours being especially preferred in certain embodiments.

The method according to the first aspect of the present disclosure further comprises a step c. of concentrating the population of genetically modified cells, thereby generating a population of concentrated cells comprising the genetically modified cells at a second concentration. According to the invention, the second concentration is higher than the first concentration.

It is preferred that the second concentration is a higher concentration at which typically the cell density effect is present in the cell culture, however, the present disclosure is not limited in this regard. Rather, it is preferred that the second concentration is high enough to have improved space-time-yields and therefore more efficient production processes. It is appreciated that a skilled person is able to determine whether CDEs are present in the cell culture, preferably by assessing the parameters outlined elsewhere herein. The research underlying the present invention has shown that the productiveness of a cell culture can be significantly improved by avoiding or mitigating the cell density effect during introduction of the nucleic acid molecule(s) and then concentrating the resulting population of genetically modified cells such that the biological product can be produced at high cell density. It has been surprisingly found that the method according to the first aspect of the present disclosure achieves maintaining or even improving the cell-specific yield, such that higher space-time-yields than those obtainable with known methods can be achieved.

In a preferred embodiment, the first concentration is a concentration at which no cell density effect is present in the cell culture, and the second concentration is a concentration at which a cell density effect would be present in the cell culture.

In particular, the second concentration may be higher than the first concentration by a concentration factor in the range of at least 1.5 up to 1000, preferably at least 2, at least 3, at least 5 or at least 10, further preferably up to 100 or up to 20.

For example, the second concentration may be in the range of 2-200 x 10⁶ cells per mL, preferably 6-100 x 10⁶ cells per mL, e.g. 9 x 10⁶ cells per mL.

In particular, an exemplary first concentration may be reached by diluting a cell seed comprising 12 x 10⁶ cells per mL down to a concentration of 3 x 10⁶ cells per mL. After introducing the one or more nucleic acid molecules at the first concentration, a second concentration of 9 x 10⁶ cell per mL may be reached by concentrating the genetically modified cells.

The concentrating in step c. may be performed by centrifugation and resuspension, filtration, and/or sedimentation. For example, the filtration may be in alternating tangential flow (ATF), or may be a tangential flow filtration (TFF).

The method according to the first aspect of the present invention further comprises the step d. of cultivating (also referred to herein as "maintaining") the concentrated cells under conditions conducive to expression of the product.

In the context of the present disclosure, the term "conditions conducive to expression" is intended to refer to a set of physicochemical and biological parameters adjusted or optimized to support cell viability, metabolic activity, and efficient transcription, translation, and post-translational processing of the biological product. These conditions correspond to those typically employed for the production of biological products in cell culture and may therefore be routinely determined by a skilled person. These conditions include, but are not limited to, appropriate temperature, pH, osmolarity, oxygenation, carbon dioxide levels, nutrient availability, and/or cell density, and may further include agitation and shear conditions suitable for maintaining cellular integrity and promoting protein synthesis. For example, HEK cells may be maintained at 37°C with 5-8% CO₂ in serum-containing or chemically defined media with sufficient glucose and glutamine. Transient expression efficiency may, for example, be enhanced by adjusting osmolality (e.g., 280-350 mOsm/kg).

For example, the cultivating may be performed in batch culture, fed-batch culture or perfusion culture, wherein perfusion culture is preferred. Cultivating the cells in perfusion culture has the advantage that the yield of biological product per bioreactor volume is higher than other cultivation systems, which is a crucial consideration for biomanufacturers. Along the same lines, biological products produced in perfusion culture typically have the advantage of having a reduced cost per gram of nucleic acid used.

The cultivation step may extend for a duration typically used in the field of biomanufacturing, e.g. up to 21 days or up to 14 days. In some embodiments, wherein the biological product is preferably a viral vector, such as an AAV vector, the duration ranges from 12 h to 96 h, preferably 48 h to 96 h. During cultivation, the cell culture medium may be exchanged at least once, e.g. by perfusion, aspiration after sedimentation, aspiration after centrifugation, or combinations thereof.

In case the cultivation is carried out in perfusion culture, the step of cultivating may comprise an initializing phase and a main cultivation phase, wherein a medium exchange rate during the initializing phase may differ (e.g., be higher) from the medium exchange rate during the main cultivation phase. For example, during the initializing phase, the medium exchange rate may be 3-8 VVD (vessel volume per day) and during the main cultivation phase, the medium exchange rate may be 0.5-2 VVD. Therein, the initializing phase may last for 4-24 hours following nucleic acid introduction. Thereby, it is ensured that toxic byproducts of the nucleic acid introduction are quickly and efficiently removed, such that the genetically modified cells are able to recover more quickly from stress associated with the nucleic acid introduction.

The method according to the first aspect may further comprise a step e. of harvesting the biological product from the cell culture. Harvesting procedures are known in the art and can be routinely adapted to the present invention.

In embodiments where the biological product is produced intracellularly - e.g. as intracellularly assembled viral particles - the harvesting typically involves separating the cell biomass from the culture supernatant, followed by cell lysis to release the intracellular contents. The cells may be lysed through physical methods (e.g., freeze-thaw cycles, sonication, or mechanical disruption), chemical agents (e.g., detergents or chaotropic salts), or enzymatic treatments, alone or in combination, under conditions that preserve the structural and functional integrity of the biological product. The lysate may then be clarified by centrifugation and/or filtration to remove cell debris prior to downstream purification.

In alternative embodiments, where the biological product is at least partially secreted into the culture medium - e.g. in the form of secreted viral particles, recombinant proteins, or metabolites - the harvesting may involve removal of the culture medium from the cells, optionally followed by clarification and concentration using techniques such as depth filtration, tangential flow filtration, precipitation, or a combination thereof. The remaining cell mass may be discarded or further processed if additional intracellular product is to be recovered.

In some embodiments, a combined harvesting strategy may be employed to recover both extracellular and intracellular fractions, particularly when the product is partially secreted or released through passive or active mechanisms (e.g., cell lysis, exocytosis, or budding). The precise method of harvesting and processing may be selected and optimized based on the nature of the host cells (e.g., mammalian, insect, or microbial), the stability and localization of the product, and the intended downstream applications.

It is generally preferred that the harvesting is performed as a terminal step upon completion of the production phase. For example, the entire culture volume may be collected, followed by separating the cells from the product-containing supernatant, e.g. by centrifugation, depth filtration, or a combination thereof. The cell pellet and/or the supernatant may then be subjected to a purification procedure as outlined above and elsewhere herein. Alternatively, harvesting may be conducted in multiple stages, wherein an initial fraction of the culture is harvested and fresh medium is added to facilitate batch-wise product collection while extending culture viability.

### Second aspect of the invention

According to a second aspect, the present invention provides a cell culturing system configured to carry out a method according to the first aspect.

Cell culturing systems are generally considered to be configured for carrying out the method according to the first aspect of the present disclosure if they comprise the following features:
- means for providing a population of cells capable of expressing the product, wherein the population comprises the cells at a first concentration,
- means for introducing one or more nucleic acid molecules encoding the product into the cells, thereby generating a population of genetically modified cells,
- means for concentrating the population of genetically modified cells, thereby generating a population of concentrated cells comprising the genetically modified cells at a second concentration,
   wherein the second concentration is higher than the first concentration;
- means for cultivating the cells under conditions conducive to expression of the product; and
- means for harvesting the product.

For example, as means for providing a population of cells capable of expressing the desired biological product, the system may include a first culture vessel (such as a bioreactor or a shake flask) or access thereto; wherein the first culture vessel is intended to harbor a cell seed or other source of a cell population. A suitable first culture vessel may be equipped with appropriate environmental controls for temperature, pH, and dissolved oxygen. The system may further include a second culture vessel (such as a bioreactor or a shake flask) intended to receive the cell population. The second culture vessel may be connected to a medium management module, which may be configured to provide fresh cell culture media while removing waste and spent media. The second culture vessel may further comprise monitoring sensors as described for the first culture vessel.

For example, as means for introducing one or more nucleic acid molecules encoding the biological product into the cells, the system may include a reagent addition module intended for ensuring precise delivery of nucleic acids and optional transfection reagents into the second culture vessel. Furthermore, the system may include mixing mechanisms such as agitation or gentle rocking to enhance the uniform distribution of nucleic acids across the cell population. Additionally, the system may incorporate resting conditions immediately following nucleic acid introduction, allowing for cellular recovery before concentration and cultivation is initiated.

For example, as means for concentrating the population of genetically modified cells, the system may comprise a concentration module intended to facilitate the concentration of cells. The concentration module may encompass means for centrifuging the cell culture, or means to filtrate the cell culture (such as an ATF filter unit).

For example, as means for cultivating the genetically modified cells, the system may comprise a bioreactor or shake flask configured to subject the cell culture to controlled conditions to maintain optimal cellular metabolism and production of the biological product, in particular controlled temperature, dissolved oxygen regulation, and/or pH control.

For example, the system may further include means for harvesting the biological product. Such means may include any combination of the following:
- means for collecting cell-free supernatant, particularly for harvesting secreted products such as proteins or viral particles,
- means for cell lysis and intracellular product recovery, particularly for product which is retained within the cells,
- means for filtration of the cell culture, such as depth filtration, tangential flow filtration (TFF), or centrifugation to remove cell debris while retaining the biological product, and/or
- means for purifying the biological product, such as chromatographic units configured for, e.g., affinity chromatography or ion-exchange chromatography.

It is preferred that the system is configured to carry out at least parts of the method according to the first aspect in an automated fashion, for example steps c. and d., preferably steps b. to d. or steps b. to e.

### Third aspect of the invention

According to a third aspect, the present invention provides a use of a cell culturing system according to the second aspect in a method according to the first aspect.

### EXAMPLES

The invention will now be described in more detail in relation to working examples and with reference to the Figures. The working examples are intended to merely illustrate some of the working embodiments of the invention and shall not be construed in a limiting fashion. The Figures show in
- Fig. 1: a schematic overview of the method according to the invention (method A) and comparative methods from the prior art (methods B and C);
- Fig. 2: a schematic overview of the media exchange rates during cultivation as part of the methods A, B and C;
- Fig. 3: the total capsid yield (capsid per mL) obtained from cultures processed according to methods A, B and C;
- Fig. 4: the total functional capsid yield (TU per mL) obtained from cultures processed according to methods A, B and C;
- Fig. 5: the total amount of capsids produced per cell (CSVY, capsids per cell) obtained from cultures processed according to methods A, B and C;
- Fig. 6: the total amount of functional capsids produced per cell (CSVY, functional capsids per cell) obtained from cultures processed according to methods A, B and C;
- Fig. 7: the space-time yield (STY; capsids per mL per day) obtained from cultures processed according to methods A, B and C;
- Fig. 8: the functional space-time yield (STY; functional capsids per mL per day) obtained from cultures processed according to methods A, B and C;
- Fig. 9: the viable cell density (cells per mL) determined in cultures processed according to methods A, B and C; and
- Fig.: 10 the cell viability (%) determined in cultures processed according to methods A, B and C.

### Example: Production of AAV particles in a perfusion bioreactor system

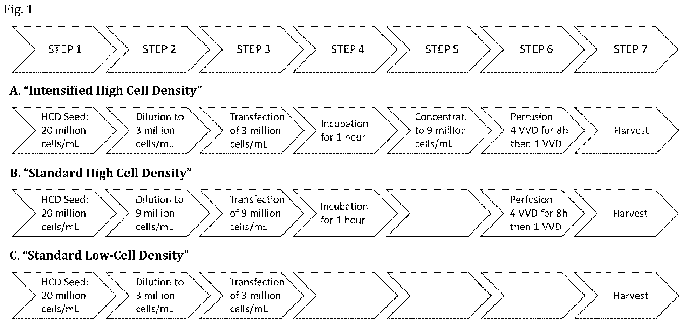

To confirm the workability and advantages of the present invention, a method according to the first aspect of the invention was carried out in comparison with two methods from the prior art. Fig. 1 shows a comparative overview of the respective methods.

**A. Method according to the invention (see** **Fig. 1A****):** A population of suspension-adapted HEK293 cells was provided from a pre-established cell seed maintained in a chemically defined, serum-free medium under controlled conditions (37°C, 5% CO₂, pH 7.0) in a perfusion bioreactor at a cell concentration of approx. 20 x 10⁶ cells per mL. The providing step included diluting the cells in fresh culture medium to a first concentration of 3 x 10⁶ cells per mL. As a nucleic acid, a recombinant AAV vector was introduced to the cell population by transfection. Transfection was carried out by preparing a transfection complex containing nucleic acid-synthetic polymeric agent polyplexes. The nucleic acid component consisted of triple transfection plasmid DNAs, which were diluted with PBS to achieve a concentration of 1 µg nucleic acid per 1 million cells. After dilution, synthetic polymeric transfection agents were added to initiate the formation of transfection complexes. Once the transfection complexes reached the required size, the mixture was added to the cells. After having been subjected to transfection, the cells were incubated for a period of 1 hour in cultivation vessels under controlled conditions (37°C, 5% CO₂, pH 7.0). Following the incubation, the cells were concentrated by centrifugation until a second concentration of 9 x 10⁶ cells per mL was reached. Cells were then cultivated in a perfusion culture. The medium exchange rate was adjusted to 4 VVD for the first 8 hours of cultivating, and was then changed to 1 VVD for the remaining duration of the cultivation process, which lasted for a total of 72 hours. The biological product was then harvested by adding a chemical lysis reagent supplemented with a nuclease into the cultivation vessel and incubating at 37°C for 1 hour. The resulting culture lysate was then subjected to a partial purification procedure using sterile filtration.

**B. High cell density (HCD) method of the prior art (see** **Fig. 1B****):** This comparative method was identical to the method of the invention except for the following details. The cells from the cell seed were diluted to a concentration of 9 x 10⁶ cells per mL. Transfection of the cells was performed at that concentration. No step of concentrating the cells was performed. The transfected cells were cultivated in perfusion culture with a starting cell concentration of 9 x 10⁶ cells per mL.

**C. Low cell density (LCD) method of the prior art (see** **Fig. 1C****):** This comparative method was identical to the method of the invention except for the following details. No step of concentrating the cells was performed. The transfected cells were cultivated in batch culture for 72 hours with a starting cell concentration of 3 x 10⁶ cells per mL.

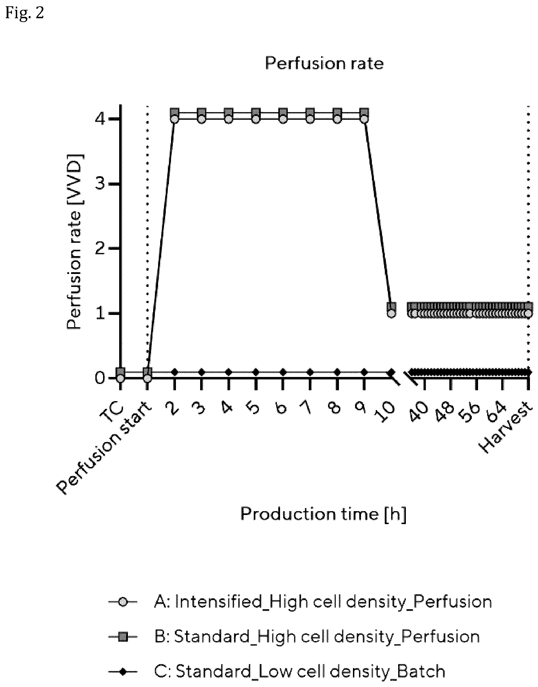

Figure 2 shows a schematic overview of the medium exchange rates during the cultivation step. As shown, methods A. and B. were carried out in perfusion culture with a medium exchange rate of 4 VVD for the first 8 hours of cultivating (until the timepoint 9 hours), after which the exchange rate was lowered to 1 VVD for the remainder of the cultivating period. Batch method C saw no medium exchange (0 VVD) over the entire cultivating period.

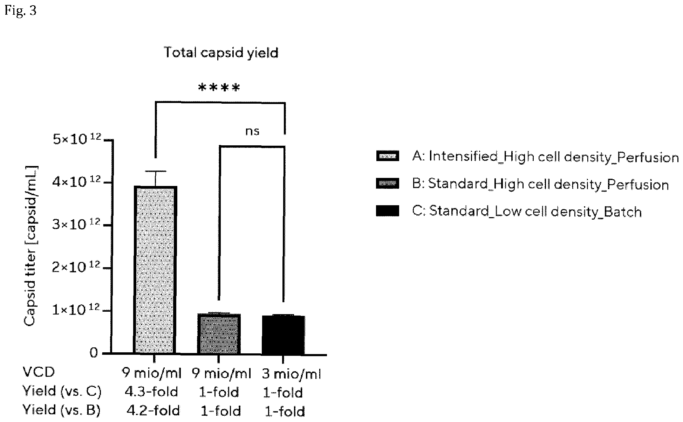

Figure 3 shows the total capsid titers obtained from the respective cultures. This value corresponds to the total number of assembled AAV capsids, regardless of whether they contain genomic material. This value corresponds to the overall productivity of each cell culture, and helps in assessing capsid assembly efficiency. This measurement was obtained using AAVX biosensors, where AAVX antibodies are immobilized. Quantification of capsid titers is achieved by determining the binding rate of the AAV vector in the test sample to the AAVX antibodies and interpolating these binding rates with a standard curve.

Approximately 4 x 10¹² capsids per mL culture volume were obtained from the culture produced by the method A. according to the invention, while the methods B. and C. from the prior art produced only approx. 1 x 10¹² capsids per mL culture volume. The method of the invention therefore resulted in an approx. 4-fold increase in capsid titer over the methods from the prior art. This difference was significant (p < 0.0001 = ****). The methods from the prior art did not differ significantly (ns). This result shows that the culture obtained by the method of the invention was considerably more productive than comparable cultures from the prior art.

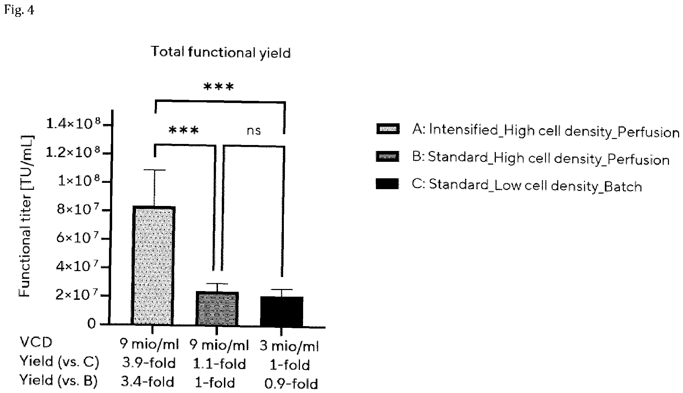

Figure 4 shows the functional capsid titers obtained from the respective cultures. This value corresponds to the subset of total capsids that contain vector genomes and are capable of delivering the contained genetic information to target cells (so-called transducing units, TU). This measurement was obtained by adding sterile serially diluted AAV test samples to adherent HEK293 cells. After a 72-hour incubation period, GFP expression in the transduced cells was analyzed using an imaging system, capturing high-resolution fluorescence and bright-field images.

Approximately 8 x 10⁷ functional capsids per mL culture volume were obtained from the culture produced by the method A. according to the invention, while the methods B. and C. from the prior art produced only approx. 2 x 10⁷ functional capsids per mL culture volume. The method of the invention therefore resulted in an approx. 3.5- to 4-fold increase in functional capsid titer over the methods from the prior art. This difference was significant (p < 0.001 = ***). The methods from the prior art did not differ significantly (ns). This result shows that the method according to the invention results in a larger number of functional particles per mL of culture volume. The percentage of functional capsids was similar between the methods, showing that the higher productivity obtained with the method according to the invention is not at the expense of capsid quality.

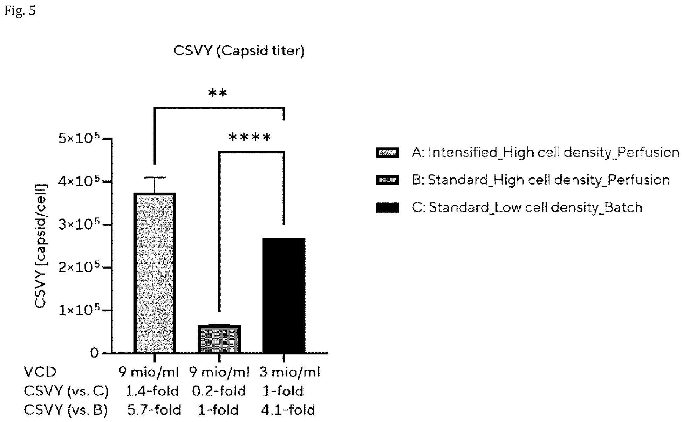

Figure 5 shows the CSVY value, i.e. the total number of capsids produced per cell during the culture period. It is calculated by dividing total capsid yield by the total number of cells. This value measures how efficiently each cell produces the capsid.

Approximately 3.9 x 10⁵ capsids per cell were obtained from the culture produced by the method A according to the invention. Method B from the prior art produced only approx. 0.7 x 10⁵ capsids per cell, and method C from the prior art produced approx. 2.8 x 10⁵ capsids per cell. The method of the invention therefore resulted in an approx. 5.7-fold increase in capsid titer over method B (p < 0.0001 = ****), and in an approx. 1.4-fold increase over method C (p < 0.01 = **). Method C led to a more favorable result than method B (p < 0.0001 = ****). This result shows that carrying out the cultivation period at high cell densities does not negatively impact the cell culture output when the transfection is performed at low cell density. Moreover, the result surprisingly shows that the method according to the invention, possibly due to the concentration step, even leads to improved productivity per cell, which is unexpected given the prior knowledge that CDE negatively affects cell productivity.

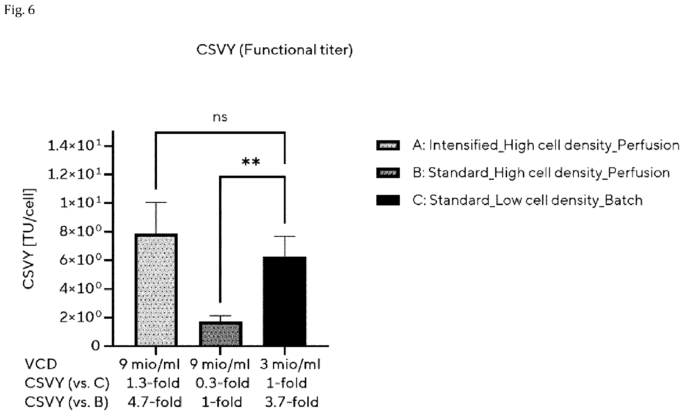

Figure 6 shows the functional CSVY value, i.e. the total number of functional capsids produced per cell during the culture period. It is calculated by dividing total functional capsid yield by the total number of cells. This value measures how efficiently each cell produces functional capsid.

Approximately 8 (x 10⁰) functional capsids per cell were obtained from the culture produced by the method A according to the invention. Method B from the prior art produced only approx. 1.7 functional capsids per cell, and method C from the prior art produced approx. 6 functional capsids per cell. The method of the invention therefore resulted in an approx. 4.7-fold increase in capsid titer over method B (p < 0.001 = ***), and in an approx. 1.3-fold increase over method C (p > 0.05 = ns). Method C led to a more favorable result than method B (p < 0.01 = **). This result shows that carrying out the cultivation period at high cell densities does not negatively impact the production output of functional virus capsids as long as the transfection is performed at low cell density.

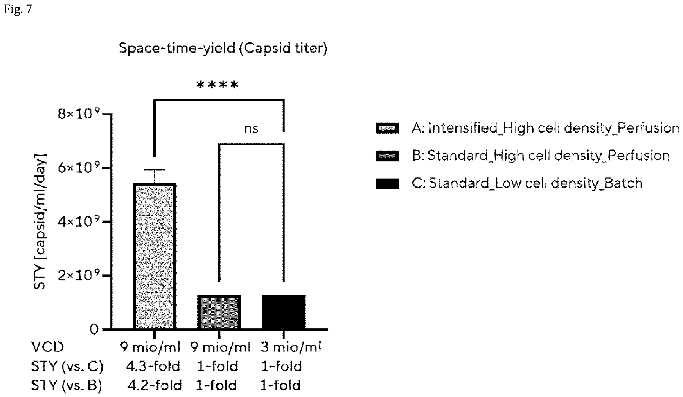

Figure 7 shows the space-time yield (STY), i.e. the amount of capsid produced per mL of culture volume per day. It is determined by dividing the capsid concentration produced per day by the reactor volume.

Approximately 5.5 x 10⁹ capsids per mL per day were obtained from the culture produced by the method A according to the invention. Method B and C from the prior art each produced only approx. 1 x 10⁹ capsids per mL per day. The method of the invention therefore resulted in an approx. 4.2- and 4.3-fold increase in space-time yield over methods B and C (p < 0.0001 = ****). This result confirms that the method according to the invention leads to an improvement in the efficiency of production over time and per unit of bioreactor volume, thus marking a considerable improvement for biomanufacturers.

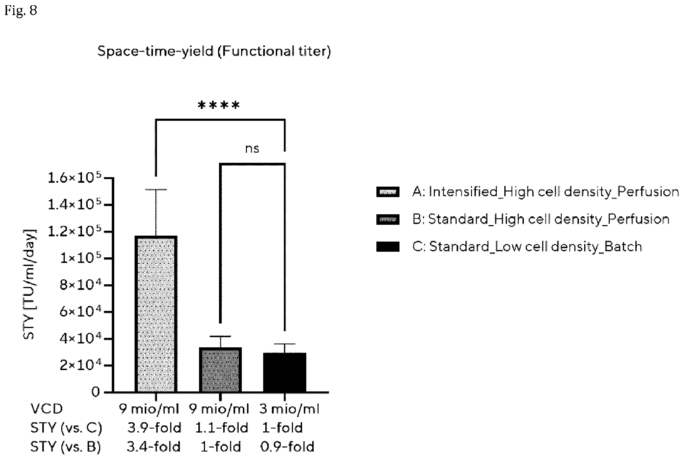

Figure 8 shows the space-time yield (STY) of functional AAV capsids, i.e. the amount of functional capsid produced per mL of culture volume per day. It is determined by dividing the functional capsid concentration produced per day by the reactor volume.

Approximately 1.2 x 10⁵ functional capsids per mL per day were obtained from the culture produced by the method A according to the invention. Method B and C from the prior art each produced between 2 and 4 x 10⁴ functional capsids per mL per day. The method of the invention therefore resulted in an approx. 3.4- and 3.9-fold increase in space-time yield over methods B and C (p < 0.0001 = ****), respectively. The methods from the prior art did not differ significantly (ns). This result confirms that the method according to the invention leads to an improvement in the efficiency of production of functional capsids over time and per unit of bioreactor volume, thus marking a considerable improvement for biomanufacturers.

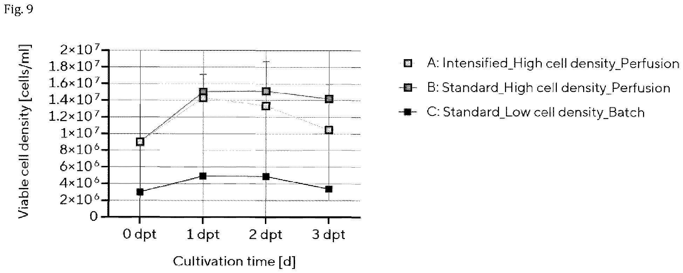

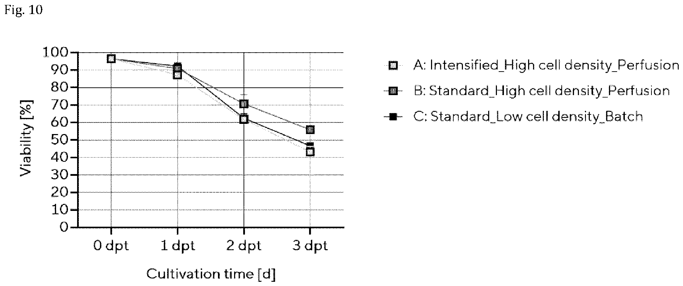

Figure 9 shows the viable cell densities and Figure 10 the cell viability obtained by methods A, B and C. Both values were measured every 24 hours, starting on the day of transfection and ending before harvesting the cell culture. Each experimental setup was conducted in duplicates, the error bars represent standard deviation from the mean (n = 2).

The highest viable cell densities were achieved by the previously known high cell density method B, where cell densities peaked at approx. 1.5 x 10⁷ cells per mL and remained above 1.4 x 10⁷ cells per mL until the end of the culturing period. Following the method according to the invention, the viable cell density decreased by approx. 30% between 1 dpt (= days post transfection) and 3 dpt to a value of approx. 1 x 10⁷ cells per mL. At the same time, the cell viability similarly decreased in all bioreactors, independent of whether method A, B or C was performed. Without wishing to be bound by theory, these results indicate that the method according to the invention leads to a reduced cell division activity, possibly due to an increased focus of the cellular bioprocessing machinery on AAV production, without negatively affecting overall cell viability when compared to known methods.

## Claims

1. Method for transiently producing a biological product in cell culture, comprising:
a. providing a population of cells, wherein the population comprises the cells at a first concentration,
b. introducing one or more nucleic acid molecules encoding the product into the cells, thereby generating a population of genetically modified cells,
c. concentrating the population of genetically modified cells, thereby generating a population of concentrated cells comprising the genetically modified cells at a second concentration,
wherein the second concentration is higher than the first concentration; and
d. cultivating the concentrated cells under conditions conducive to expression of the product.

2. The method according to claim 1, wherein the first concentration is a concentration at which no cell density effect is present in the population of cells.

3. The method according to claim 1 or 2, wherein the second concentration is higher than the first concentration by a concentration factor in the range of at least 1.5 up to 1000, preferably at least 2, at least 3, at least 5 or at least 10, further preferably up to 100 or up to 20.

4. The method according to any one of the preceding claims, wherein the first concentration is in the range of 0.01-6 x 10⁶ cells per mL, preferably 0.5-6 x 10⁶ cells per mL, more preferably 1-3 x 10⁶ cells per mL, e.g. 3 x 10⁶ cells per mL, and/or
wherein the second concentration is in the range of 2-200 x 10⁶ cells per mL, preferably 6-100 x 10⁶ cells per mL, e.g. 9 x 10⁶ cells per mL.

5. The method according to any one of the preceding claims, wherein the cells are eukaryotic cells, preferably mammalian or insect cells, further preferably wherein the cells are selected from the group consisting of HEK and CHO cells, further preferably wherein the cells are HEK cells.

6. The method according to any one of the preceding claims, wherein the one or more nucleic acid molecules encode a viral vector, preferably selected from the group consisting of adenoviral vector, lentiviral vector, AAV vector, baculoviral vector, HSV vector, vaccinia virus vector, and SFV vector,
more preferably wherein the viral vector is an AAV vector.

7. The method according to any one of the preceding claims, wherein the biological product is an intact virus, wild-type or recombinant, capable of infecting a target cell, preferably wherein said virus retains its full structural and functional integrity, including its capsid and a polynucleotide.

8. The method according to any one of the preceding claims, wherein in step a., the population of cells is provided by:
- providing a cell seed comprising the cells at a seeding concentration which optionally differs from the first concentration, and
- adjusting the cell concentration of the cell seed until the first concentration of the cells is reached,
preferably encompassing an exchange of cell culture media,
preferably wherein the cell seed is obtained from a continuous seed system.

9. The method according to any one of the preceding claims, wherein prior to step b., the population of cells is maintained for a period of time, preferably wherein the period of time is at least 10 minutes, at least 15 minutes, at least 20 minutes, at least 25 minutes, at least 30 minutes, e.g. up to 4 hours, up to 2 hours or up to 1 hour.

10. The method according to any one of the preceding claims, wherein at the end of step b., the cells are incubated for a period of time, preferably wherein the period of time is at least 10 minutes, at least 15 minutes, at least 20 minutes, at least 25 minutes, at least 30 minutes, e.g. up to 4 hours, up to 2 hours or up to 1 hour.

11. The method according to any one of the preceding claims, wherein the concentrating in step c. is performed by centrifugation and resuspension, filtration, and/or sedimentation.

12. The method according to any one of the preceding claims, wherein in step d., the cultivating is performed in batch culture, fed-batch culture or perfusion culture, preferably perfusion culture.

13. The method according to any one of the preceding claims, further comprising step e. of harvesting the biological product.

14. Use of a cell culturing system to carry out a method according to any one of the preceding claims, the system comprising:
- means for providing a population of cells capable of expressing the biological product, wherein the population comprises the cells at a first concentration,
- means for introducing one or more nucleic acid molecules encoding the biological product into the cells, thereby generating a population of genetically modified cells,
- means for concentrating the population of genetically modified cells, thereby generating a population of concentrated cells comprising the genetically modified cells at a second concentration,
wherein the second concentration is higher than the first concentration;
- means for cultivating the cells under conditions conducive to expression of the biological product; and
- optionally means for harvesting the biological product.

15. The use according to claim 14, wherein the system is configured to carry out at least steps c. and d., preferably steps b. to d., of the method according to any one of claims 1 to 13 in an automated fashion.
